# EUROPEAN PATENT APPLICATION

(11) **EP 1 611 896 A1**
(43) Date of publication of application: **04.01.2006**
(21) Application number: 04723965.2
(22) Date of filing: 29.03.2004
(51) Int. Cl.: A61K 31/7008, A61P 37/06

(54) **USE OF N-ACETYL-D-AMINOGLYCOSAMINE IN TREATMENT OF LOCAL LESIONS OR SYSTEMATIC SYMPTOMS RELATED TO AUTOIMMUNE REACTIONS**

(30) Priority: 27.03.2003 CN 03108277
(71) Applicant: Third Military Medical University Chinese People's Liberation Army P.R. of China, Chongqing 400038 (CN); Beijing Sino-Hongkong Dafu Science & Technology of Biowave Co., Ltd., Beijing 101200 (CN); Bio-Wave Institute of Suzhou Hi-Tech New District Corporation, Ltd, Jiangsu 215011 (CN)
(72) Inventor: XU, Qiwang, Chongqing 400038 (CN); LIU, Junkang, Chongqing 400038 (CN); YUAN, Zetao, Chongqing 400038 (CN)
(74) Representative: Carpintero Lopez, Francisco
(86) International application number: PCT/CN2004/000275
(87) International publication number: WO 2004/084913

(57) **Abstract**

The present invention discloses a method for treating local lesions or systematic symptoms caused by autoimmune reactions, a use of N-acetyl-D-glucosamine for treating local lesions or systematic symptoms caused by autoimmune reactions, and a use of N-acetyl-D-glucosamine in the manufacture of a medicament for treating local lesions or systematic symptoms caused by autoimmune reactions.

## Description

### Technical field

The present invention relates to the use of N-acetyl-D-glucosamine or pharmaceutically acceptable salts thereof in the treatment of local lesions and/or systematic symptoms caused by autoimmune reactions, and the use of N-acetyl-D-glucosamine or pharmaceutically acceptable salts thereof in the manufacture of a medicament for the treatment of local lesions and/or systematic symptoms caused by autoimmune reactions.

### Background Art

At present, there are two main methods for treatment of systematic symptoms, such as fever, headache, vertigo, delirium, nausea, emesis, general malaise, etc., caused by autoimmune reactions: 1) immune suppression therapy by using cortisone, etc.; and 2) supporting therapy. Since the use of hormones may result in many side-effects, the supporting therapy is usually used at present, but the effect of the supporting therapy is not satisfactory. Thus, drugs for the treatment of local lesions and/or systematic symptoms caused by autoimmune reactions are still in need.

In the research of "bio-waves" theory, the present inventor has set up a organism wave-growth model. Through deeply researching the molecular mechanism of the organism wave-growth, the inventor puts forward a micro-heterology variation mechanism, wherein the biological wave of organism continuously changes; the change rate depends on the change extent of outer environments; after the organism is wounded so that its antigens are exposed, or the substances of the organism are denatured due to other factors, the function of immunological cells changes, which promotes the generation of micro-heterology and causes local lesions and systematic symptoms associated to autoimmune reactions.

It is found that N-acetyl-D-glucosamine as a regulating factor of biological wave affects not only the macro fluctuation, but also the stability of vibration of macromolecular substances. This substance can maintain the physiological vibration of macromolecular substances, alleviate and repulse harmful effects in organism, in order to maintain the physiological function of macromolecular substances and to avoid complications caused by autoimmune reactions. In general, said substance can control conditions caused by autoimmune reactions, alleviate lesions, promote heal, and eliminate conditions.

The inventor surprisingly finds that N-acetyl-D-glucosamine or pharmaceutically acceptable salts in combination with various pharmaceutically acceptable carriers can form suitable formulation forms for treatment of local lesions or systematic symptoms caused by autoimmune reactions, so as to carry out the present invention.

### Contents of the invention

One object of the present invention is to prove a use of N-acetyl-D-glucosamine or pharmaceutically acceptable salts thereof in the treatment and control of local lesions and systematic symptoms caused by autoimmune reactions.

Another object of the present invention is to provide a use of N-acetyl-D-glucosamine or pharmaceutically acceptable salts thereof in the manufacture of a medicament for the treatment and control of local lesions and systematic symptoms caused by autoimmune reactions.

Another object of the present invention is to provide a method for treating local lesions and systematic symptoms caused by autoimmune reactions.

The N-acetyl-D-glucosamine used in the present invention is a compound having a molecular formula of C₈H₁₅NO₆ and a structure formula (I).

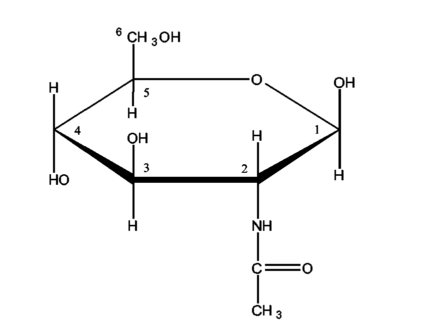

The examples of pharmaceutical acceptable salts of N-acetyl-D-glucosamine that can be used in the present invention include, but are not limited to: the salts formed with inorganic acids, such as hydrochloride, hydrobromide, borate, phosphate, sulfate, hydrosulfate and hydrophosphate, and the salts formed with organic acids, such as citrate, benzoate, ascorbate, methylsulfate, picrate, fumarate, maleate, malonate, succinate, tartrate, mesylate, and glucose-1-phosphate.

In a pharmaceutical composition of the present invention, the content of N-acetyl-D-glucosamine or pharmaceutically acceptable salts thereof is generally 0.1-10% by weight.

Besides N-acetyl-D-glucosamine or pharmaceutically acceptable salts thereof, said pharmaceutical composition further comprises excipients or carriers well known in the art to form a preparation suitable for intravenous injection, subcutaneous injection, intramuscular injection, intraperitoneal injection, etc., or to form preparations suitable for oral administration.

Said pharmaceutical composition can be administered in a manner of single dose per day or multidoses per day, such as 3-4 doses per day. The dose of said pharmaceutical composition depends on patient's age, condition, symptom, and administration manner. In general, as to an adult patient having a bodyweight of 75 kg, the dose of said pharmaceutical composition is 1-100000 mg per day, based on active component, and is administered one to four times daily.

According to a preferable model, N-acetyl-D-glucosamine is administered in a manner of intervenous drop infusion during the therapeutical procedure in order to potentiate power of resistance, to replenish water, and to maintain stability in vivo.

As compared to conventional supporting therapy, N-acetyl-D-glucosamine or pharmaceutically acceptable salts thereof are more effective in reducing local inflammations and alleviating local lesions and systematic toxic symptoms, act quickly and roundly, and facilitate better prognostic results.

### Embodiments for carrying out the invention

The beneficial effects of the present invention are further demonstrated by the following examples, but it shall be understand that these examples are merely to illustrate the present invention, rather than to restrict the scope of the present invention in any aspect.

### Example 1. Promoting wave test of the compound of formula (I)

### 1. Experimental materials and method:

1.1 Samples: pure compound of formula (I)
1.2 Experimental materials:
   Strain: *Proteus Mirabilis* that meets the following biochemical reaction characteristics: dynamics (+), urease (+), lactose (-), glucose (+), H₂S (-), phenylalanine deaminase (+).
   Culture medium: modified LB culture medium (components: 1% tryptone, 0.5% yeast extract, 1% sodium chloride, 0.1 % glucose, 0.002% TTC, and pH = 7.2 to 7.4).
1.3 Experimental method:
   Control sample: the *Proteus Mirabilis* were inoculated at the center of LB plate, incubating at 37°C for 9 hours;
   Test sample: the compound of formula (I) with a final concentration of 0.5% was added to the LB plate, then the *Proteus Mirabilis* were inoculated by the same method, and cultured at 37°C for 9 hours.

### 2. Experimental results and evaluation:

The control sample exhibited concentric rings with an interval of 3 hours, which extended outward continually. The test sample showed not only concentric rings with an interval of 3 hours, but also many fine waves on each ring in comparison with the control sample.
The experiment adopts a bio-wave model which is used to research the promoting wave function of the compound of formula (I). The results showed that the compound of formula (I) was not only able to cause bacterial cell to reveal a normal bio-wave characteristic, but also cause the wave reveal finer wave mode. These indicated that the compound of formula (I) has a function of promoting bio-waves. This wave-promoting function may explain the effects of using N-acetyl-D-glucosamine or pharmaceutically acceptable salts thereof for treating and controlling local lesions and systematic symptoms caused by autoimmune reactions.

### Example 2. Toxicological test of the compound of formula (I)

The toxicological test of the compound of formula (I) includes:
1. Acute toxicity test: including tests of oral administration, intravenous injection administration, and maximum limit amount for administration;
2. Ames test;
3. Micronucleus test of mouse bone marrow cell;
4. Abnormality test of mouse sperm;
5. Aberration test of mouse testis chromosome;
6. Chronic lethal test;
7. Sub-chronic toxicity (feed for 90 days) test;
8. Traditional deformity-inducing test.

The results of these tests showed that in the acute toxicity test of the compound of formula (I), the acute toxicosis reaction had not appeared when the dosage more than 2 g/kg was taken; in the long-period toxicity test, the maximum dosage had reached up to 1 g/kg, and after the treatment and observation for four weeks, there was no intoxication reaction yet; and in the reproduction test, the mice were feed with a routine dosage of 7 mg/kg for 3 generations, it had been proved that the compound of formula (I) had no influence on the pregnancy, birth, nurse and the growth of baby mouse, so that the compound of formula (I) is a substance without toxicity.

### Example 3. Cytological tests of regulating micro-heterology variation

Conventional incomplete 1640 culture media were used for cell culture, and B16 tumor cells (commercially obtained from the tumor cell library of Shanghai Institute of Cytobiology) were inoculated on said media. After continue culture for more than 48 hours, the micro-heterology variation of cells and the control effects of N-acetyl-D-glucosamine thereon were observed under a condition where metabolic wastes affected the growth environment. After N-acetyl-D-glucosamine having a final concentration of 1 g/100ml was added to the culture media, the cell number stably increased with the culture time during the cell growth procedure. The control cells cultured without N-acetyl-D-glucosamine could not proliferate on the same culture media under same conditions. These tests indicated that in the presence of the compound of formula (1), cells could regulate the cell micro-heterology variation in order to adapt to the ever-changing environment, so that cells could proliferate continuously.
When N-acetyl-D-glucosamine was replaced with N-acetyl-D-glucosamine hydrochloride in the tests (other conditions were not changed), as compared to the control test, cells also could regulate the cell micro-heterology variation in order to adapt to the ever-changing environment, so that cells could proliferate continuously.

### Example 4. Animal tests that N-acetyl-D-glucosamine regulates micro-heterology variation

B16 tumor cells were inoculated on superior parts of hind legs of 50 rats, and 5% aqueous solution of the compound of formula (1) was intra-peritoneally injected to the rats for consecutive 7 days, 3 times per day, and 1ml every time. Finally, solid tumor did not appear in 45 rats. In control group without the administration of the compound of formula (1), many proliferative corpuscles appeared in at least 40 among 50 rats within 1-3 days after the tumor cells were inoculated; many immature cells appeared within 3-5 days; and visible solid tumors finally appeared within about 10 days. As compared to the control group, this did not appear in the test group, which indicated that the compound of formula (1) could control the micro-heterology variation.
When N-acetyl-D-glucosamine was replaced with N-acetyl-D-glucosamine hydrochloride in the tests (other conditions were not changed), as compared to the control group, N-acetyl-D-glucosamine hydrochloride also exhibited function of controlling micro-heterology variation.

### Example 5. Use of N-acetyl-D-glucosamine for treatment of systemic lupus erythematosus

Patient, Mrs. Liu, who had definite systemic lupus erythematosus, was administered with capsules of the compound of formula (1) for consecutive 7 days, 3 times per day, and 100 mg every time, during the period of hospitalization. The patient's symptom was remitted in some extent, and the serum titer of antinuclear antibody decreased from 1:160 to 1:40 that was close to normal level.

### Example 6. Use of N-acetyl-D-glucosamine for treatment of hyperthyroidism

Patient, Mr. Shen, was administered with capsules of the compound of formula (1) for consecutive 7 days, 3 times per day, and 100 mg every time, during the period of treating diarrhea. It was surprisingly found that said compound exhibited better effect of treating hyperthyroidism, and the therapeutical effect of the compound of formula (1) for treatment of hyperthyroidism was confirmed by tests of T3 and T4 in serum.

## Claims

1. A use of N-acetyl-D-glucosamine or pharmaceutically acceptable salts thereof in treating and controlling local lesions and systematic symptoms caused by autoimmune reactions.

2. A use of N-acetyl-D-glucosamine or pharmaceutically acceptable salts thereof in the manufacture of a medicament for treating and controlling local lesions and systematic symptoms caused by autoimmune reactions.

3. A use according to claim 2, wherein said medicament is a preparation suitable for intravenous injection, subcutaneous injection, intramuscular injection, intra-peritoneal injection, or oral administration.

4. A use according to 2 or 3, wherein the dose of said medicament for an adult patient is 1-100000 mg per day based on the active component, and said medicament is administered one to four times daily.

5. A method for treating and controlling local lesions and systematic symptoms caused by autoimmune reactions, wherein a pharmaceutical composition comprising an effective amount of N-acetyl-D-glucosamine or pharmaceutically acceptable salts thereof is administered to a patient.

6. A method according to claim 5, wherein said pharmaceutical composition is a preparation suitable for intravenous injection, subcutaneous injection, intramuscular injection, intra-peritoneal injection, or oral administration.

7. A method according to 5 or 6, wherein the dose of said pharmaceutical composition for an adult patient is 1-100000 mg per day based on the active component.
